(19) Europäisches Patentamt
European Patent Office
Office européen des brevets

(11) Publication number: 0 040 898
B1

(12) # EUROPEAN PATENT SPECIFICATION

(45) Date of publication of patent specification: **16.01.85**

(51) Int. Cl.$^4$: **C 07 C 79/46,** A 01 N 37/48

(21) Application number: **81300348.0**

(22) Date of filing: **27.01.81**

(54) **2-Nitro-5-(substituted-phenoxy) benzoate esters of alpha-hydroxyalkanoates and methods of using same as herbicides.**

(30) Priority: **23.05.80 US 152876**

(43) Date of publication of application:
**02.12.81 Bulletin 81/48**

(45) Publication of the grant of the patent:
**16.01.85 Bulletin 85/03**

(84) Designated Contracting States:
**AT BE CH DE FR IT LI NL SE**

(56) References cited:
**EP-A-0 020 052**
**DE-A-2 311 638**
**DE-A-2 753 900**

(73) Proprietor: **RHONE-POULENC AGROCHIMIE**
**14-20, rue Pierre Baizet**
**F-69009 Lyon (FR)**

(72) Inventor: **Downing, Charles Rupert**
**91 Amboy Avenue**
**Metuchen New Jersey 08840 (US)**
Inventor: **Theissen, Robert James**
**474 Van Holten Road**
**Bridgewater New Jersey 08807 (US)**
Inventor: **Holm, Robert Eric**
**27 Kingswood Drive**
**Belle Mead New Jersey 08502 (US)**

(74) Representative: **Brachotte, Charles et al**
**RHONE-POULENC AGROCHIMIE 14-20, Rue**
**Pierre Baizet B.P. 9163**
**F-69263 Lyon Cedex 09 (FR)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 040 898 B1

## Description

This invention is concerned with 2-nitro-5 (substituted-phenoxy) benzoate esters of $\alpha$-hydroxyalkanoates and the methods by which they can be used as herbicides.

"US Patent No 3652645, 3784635, 3873302, 3983168 and 3907866, european patent application 20052 and German patent application 2311638 and 2753900 relate to certain derivatives which contain a diphenylether radical and to certain phenoxy benzoic acid derivatives; their utility as herbicide has been disclosed.

However it is known that herbicidal effectiveness of a diphenylether cannot be predicted from its structure only and often quite closely related compounds will have quite different weed control abilities.

We have now discovered further phenoxy benzoic acid derivatives which are useful as herbicides."

This invention relates to certain herbicidal compounds and to a method for providing season-long selective weed control.

More precisely, this invention relates to certain herbicidally active $\alpha$-hydroxyalkanoate compounds falling within the general formula:

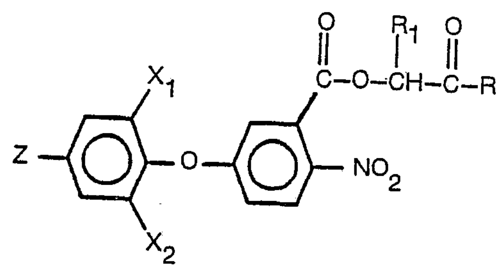

wherein

$X_1$ is a halogen;

$X_2$ is selected from the group consisting of halogen and hydrogen;

Z is a polyhalo$_{1-9}$ alkyl$_{1-4}$ group;

$R_1$ is selected from the group consisting of hydrogen and an alkyl group of 1—4 carbon atoms; and

R is alkoxy of 1—4 carbon atoms,

the following racemic compounds being excluded:

first: $Z = CF_3$; $X_1 = Cl$; $X_2 = H$; $R_1 = H$; R = tertiobutyl oxy
second: $Z = CF_3$; $X_1 = Cl$; $X_2 = H$; $R_1 = CH_3$; R = methoxy
third: $Z = CF_3$; $X_1 = Cl$; $X_2 = H$; $R_1 = CH_3$; R = ethoxy
fourth: $Z = CF_3$; $X_1 = Cl$; $X_2 = H$; $R_1 = H$; R = ethoxy
fifth: $Z = CF_3$; $X_1 = Cl$; $X_2 = H$; $R_1 = H$; R = methoxy
sixth: $Z = CF_3$; $X_1 = Br$; $X_2 = Cl$; $R_1 = H$; R = ethoxy
seventh: $Z = CCl_3$; $X_1 = I$; $X_2 = H$; $R_1 = H$; R = methoxy
eighth: $Z = CF_3$; $X_1 = Cl$; $X_2 = Br$; $R_1 = H$; R = ethoxy

This invention relates also to a method for providing season-long selective weed control by means of post-emergence and pre-emergence herbicidal effects through a single-season treatment to the cultivated area to be controlled by applying to the area a composition containing a herbicidally effective amount of a compound having the formula:

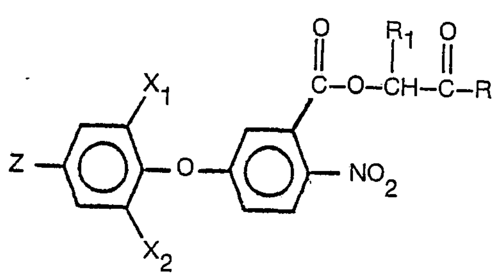

wherein:

$X_1$ is a halogen;

$X_2$ is selected from the group consisting of halogen and hydrogen;

Z is a polyhalo$_{1-9}$ alkyl$_{1-4}$ group;

$R_1$ is selected from the group consisting of hydrogen and an alkyl group of 1—4 carbon atoms; and

R is alkoxy of 1—4 carbon atoms,

the application of this effective amount being made by means of a single treatment, at rates of 0.14 to 0.84 kg/hectare, which is a post-emergence treatment for the crop and some weeds and which is a pre-emergence treatment for other late germinating weeds.

In the foregoing formula, when $R_1$ is hydrogen, the compound is referred to as a glycolate or glycolate derivative. When $R_1$ is a methyl group, the compound is referred to as a lactate or lactate derivative. Unless otherwise specified, where $R_1$ is an alkyl group, both stereoisomers are contemplated.

However, the compounds used in the present invention have very important distinguishing properties as compared with known phenoxy benzoyl compounds. One distinctive feature of the compounds of the present invention relates to their unexpected selective herbicidal properties which are not appreciably affected by the manner in which they are applied to the area to be cultivated and treated. This enables the $\alpha$-hydroxyalkanoate compounds used in the present invention to be used in a manner which provides selective herbicidal effects previously unmaintainable. These features are particularly advantageous today in view of recent developments in new techniques in farming and horticulture.

The farmer or any other grower of useful and desirable plants, the maximize the yield of desirable plants or crops, must control certain undesirable vegetation which grows in the immediate environment of the desirable plants or crops. In general, the undesirable vegetation (weeds) is detrimental because it competes with the crop for the available water, light and nutrients for crop growth as well as making management of the crop area more difficult. Weed control is generally achieved by applying certain herbicidally active compositions to the environment of the crops. To be an effective herbicide, the active ingredient of the herbicidal composition must be safe for the crops, e.g. have selectivity properties which provide a high tolerance or low phytotoxicity to the crops while at the same time effectively controlling the growth of weeds.

The ideal herbicide is one which provides selective weed control for the full growing season of the crop by a single treatment or application of the herbicide.

A significant shortcoming of many herbicides used today is that they are not effective in controlling weed growth during the full crop growing season. This can be illustrated by referring to certain other 2-nitro-5-substituted phenoxy benzoyl compounds which have carried a recommendation by the manufacturer for use in primarily one mode of application rather than another, e.g., by either pre-emergence/preplant incorporation or by post-emergence application.

These recommendations are generally founded upon practical factors, such as whether the compound achieves an effective degree of herbicidal activity against the weeds to be controlled while maintaining tolerance to the crop at rates of application which are economically feasible. In general, these compounds do not have the same herbicidal activity and/or crop tolerance when applied by pre-emergence/preplant incorporation techniques as compared with post-emergence application.

It has recently been recognized that pre-emergence and post-emergence herbicides have shortcomings in achieving overall selectivity and control of weeds. One aspect to this problem involves the inability of a single treatment or application of a herbicide to control the growth of those weeds which germinate late in the growing season. This can be illustrated by the particular agronomic aspects in soybean farming.

Soybean crops are particularly affected by late emerging weeds. Small seeded weeds usually germinate and emerge fairly uniformly in the upper inch of soil and thus are more easily controlled by pre-emergence herbicides or by a single post-emergence treatment. However, large seeded weeds germinate erratically from various depths at various times during the season resulting in "flushes" or sequential generations of weeds during the growing season. In addition to the broad-leaf weeds that germinate erratically in soybean environs, there are similar problems concerning grass weeds which escape control from pre-emergence herbicides.

The compounds used in the present invention when applied as post-emergence herbicides offer the unique ability to control later emerging weeds or weed flushes from the same early application used to kill the first flush of weeds. This is possible because these compounds exhibit pre-emergence control at post-emergence rates of 0.14 to 0.84 kg/hectare. The single treatment control achieved by the $\alpha$-hydroxyalkanoate compounds herein is a particular advantage when one considers in detail the practical problems associated with post-emergence herbicides particularly when they are applied late in a growing season.

The ultimate effectiveness of a herbicide can be a function of the technique by which it is applied to the crop/weed area. This is because herbicidal activity and crop tolerance involve various physical factors as well as chemical factors. For example, the relative physical characteristics of the emerged weed plant and crop plant can be significant. Selectivity is improved where a herbicide is applied post-emergence to weeds which have leaves of larger, rougher surfaces than the leaves of the associated crop, such as in cereal plants, which have narrow smooth leaves. In such a case, a spray of herbicide

would tend to run-off the leaves of the crop while more effectively wetting the leaves of the weeds. However, if the crop had the larger, rougher leaves as compared with those of the weeds, the effectiveness of a post-emergence herbicide could be significantly reduced.

Recently developed agricultural practices have accentuated the importance of the method by which a herbicide is applied. In soybean farming there has been a general shift to planting soybeans in narrower rows. The practice of planting 90 cm. rows is shifting to narrower rows of 35 to 50 cm. while drilled soybeans are now used in rows as narrow as 20 cm. The reason for this shift is increased yields of the soybean crop. However, a major drawback of this practice is the difficulty for growers to cultivate such narrow rows and to control weeds that escape from pre-emergence herbicides. Narrow row soybeans shade or close the rows more rapidly than conventionally planted soybeans, making the weeds that escape control from pre-emergence herbicides very difficult to reach by subsequent over-the-top post-emergence application.

Each of the foregoing problems associated with application of post-emergence herbicides is alleviated by the use of the $\alpha$-hydroxyalkanoate compounds of the present invention. A pre-emergence or early post-emergence application of the $\alpha$-hydroxyalkanoate compounds herein provides a significant residual pre-emergence activity to control later weed flushes.

The $\alpha$-hydroxyalkanoate herbicides herein used in both pre-emergence and post-emergence applications can provide existing and subsequent pre-emergence weed control. This would offer the advantage of keeping the group area free of weeds for a longer time period. This longer control may be enough to allow soybean plants to sufficiently shade the rows to reduce late season weed growth and reduce or eliminate the need to cultivate for control of escape weeds.

The increasing cost of energy to operate farm machinery and the need to conserve soil moisture and reduce soil erosion has led to the development of minimum and no-tillage agriculture. In the latter practice the ground is not plowed and seeds are planted directly into a mulch or previous crop stubble with specialized equipment in a one pass operation. This practice is heavily dependent upon the use of herbicides to kill existing vegetation (post-emergence) and to provide pre-emergence weed control so the crop can establish itself without cultivation. Present herbicide availability dictates the use of combinations of herbicides to control the existing weeds post-emergence and control germinating weeds pre-emergence. The unique post-emergence activity with subsequent pre-emergence control of the $\alpha$-hydroxyalkanoate compounds herein is particularly suitable for such minimum no-tillage agriculture technique.

A still further advantage of the $\alpha$-hydroxyalkanoate compounds herein is their flexibility for herbicidal effectiveness in both soybean and corn farming. There is a growing practice in many parts of the country to apply herbicides prior to planting using a custom applicator with large equipment. This allows the farmer to treat large acreages in a short period. However, sometimes weather conditions or other factors change and the farmer would like to switch from corn to soybeans. Because the $\alpha$-hydroxyalkanoate compounds herein are tolerant to both corn and soybean, the use of these compounds allows a shift from corn to soybeans even after herbicide application.

Herbicide compositions containing the $\alpha$-hydroxyalkanoate compounds of the present invention appear to be consistently effective in most soil types and under various environmental conditions. This is an important advantage over a number of conventional herbicides which require higher rates of application to achieve herbicidal effectiveness in some soil types, e.g., soils which contain a high organic matter content. The $\alpha$-hydroxyalkanoate compounds used in the present invention also require minimal moisture in the soil for herbicide activation.

The compounds used in this invention can be applied in various ways to achieve herbicidal action. They can be applied per se as solids or in vaporized form, but are preferably applied as the toxic components in herbicidal compositions of the compounds and a carrier thereof. These compositions are preferably applied directly to the soil and often incorporated therewith. The compositions can be applied as granulars or dusts, as liquid sprays, or as gas-propelled sprays and can contain, in addition to a carrier, additives such as emulsifying agents, binding agents, gases compressed to the liquid state, odorants, stabilizers, and the like. A wide variety of liquid and solid carriers can be used. Non-limiting examples of solid carriers include talc, bentonite, diatomaceous earth, pyrophylite, fullers earth, gypsum, flours derived from cotton seeds and nut shells, and various natural and synthetic clays having a pH not exceeding about 9.5. Non-limiting examples of liquid carriers include water, organic solvents such as alcohols, ketones, amides and esters, mineral oils such as kerosene, light oils and medium oils and vegetable oils such as cottonseed oil.

The pesticidal compositions comprising the active ingredient and the carrier may be supplied either as ready-to-use compositions or as concentrates. Concentrates generally contain a higher proportion of the active ingredient than the ready-to-use compositions and accordingly, require dilution prior to use. Both the ready-to-use compositions and the concentrates may be in liquid or solid form, i.e. with the active ingredient blended with a liquid or solid carrier, respectively.

The amount of active ingredient in the composition will depend, primarily, upon whether the composition is a concentrate or a ready-to-use composition. Concentrates will generally contain from 5 to 95% by weight, preferably 10 to 80% by weight, of the active ingredient. The concentration of active ingredient in the ready-to-use compositions will vary according to the method of application for the

composition in question and to the desired application rate. In general, ready-to-use compositions will contain from 0.001 to 1, preferably 0.01 to 0.1 percent by weight of the active ingredient. Thus, the compositions may contain from 0.001 to 95% by weight of active ingredient, the actual amount depending upon the nature of the composition and the method by which it shall be applied.

Concentrates may be either liquid or solid and are usually extendable with water to form emulsions or suspensions containing a smaller proportion of the active ingredient. Alternatively, liquid or solid concentrates may be extended with liquid or solid carriers to give the final ready-to-use composition. Liquid concentrates preferably comprise the active ingredient and an emulsifying agent dissolved in a liquid solvent e.g. an organic solvent of the type mentioned above. The emulsifier is suitably an anionic, cationic or non-ionic emulsifier, e.g. sodium dodecylbenzenesulfonate or an ethylene oxide derivative of an alkyl phenol, a mercaptan, an amine or a carboxylic acid. Liquid concentrates may advantageously contain from 10 to 30 weight percent e.g. 25 weight percent of the active ingredient e.g. 1 kg. of active ingredient per 4 kg. of concentrate.

Wettable powders are another preferred form of concentrate and these suitably comprise the active ingredient, a finely-divided solid carrier and at least one surfactant to impart wettability or dispersability. Solid carriers which are suitable for preparing wettable powder formulations may be either organic or inorganic in nature. Suitable organic carriers are soybean, walnut, or wood flour or tobacco dust, and suitable inorganic ones are clays of the bentonite, kaolinite, or fuller's earth types; silicas such as diatomaceous earth; silicates such as talc, pyrophyllite, or alkaline earth silicates; and calcium and magnesium carbonates. The carrier may be a single substance or a mixture of finely divided solids. A surfactant or mixture of surfactants is generally present in an amount of 1 to 10 percent by weight of the wettable powder formulation. Suitable dispersing agents are sodium formaldehydenaphthalene sulfonate or sodium lignin sulfonate. Wetting agents include higher alkylaryl sulfonates ("higher alkyl" meaning alkyl of at least 8 carbon atoms) such as calcium dodecylbenzenesulfonate, alcohol sulfates, alkylphenoxyethoxyethoxyethyl sodium sulfonates, sodium dioctyl sulfosuccinate, and ethylene oxide adducts with fatty alcohols, fatty acids, or with higher alkyl-phenols, such as octylphenoxypolyethoxyethanol. Sticking or spreading agents may be included such as glycerol mannitan laurate or a condensate of polyglycerol and oleic acid modified with phthalic anhydride. The active ingredient content of the wettable powder may be in the range of 20 to 80% by weight; however, the preferred range of concentrates is 50% to 80%.

Dusts may be made by incorporating the active ingredient into a solid carrier, such as finely powdered clays, talc, silica, and synthetic silicates, alkaline earth carbonates, and diluents of natural origin such as tobacco dust or walnut shell flour. Granular formulations can be made from similar type solid carriers except that the particle size is larger, in the range of 15 to 60 mesh (U.S. Standard Sieve Series). A small amount of dispersing agent may be incorporated in these solid formulations. The concentration of active ingredients in these dust or granular formulations may be in the range of 1 to 20% by weight. The solid carriers used in these formulations may be essentially inert or they may consist wholly or in part of fertilizing materials such as ammonium sulfate, or other ammonium salts, urea, calcium phosphates, potassium chloride or dried blood.

One particularly convenient method for making solid formulations is to treat the solid carrier with the active ingredients dissolved in a solvent and allow the solvent to evaporate off. This results in the carrier which is usually in the form of finely divided particles, being impregnated with the active ingredients. Another method is to apply the mixture of active ingredients in the molten state or by spraying.

The concentration of the active ingredient in the final ready-to-use composition will depend not only upon the application rate desired (generally in the range of 0.2 to 10 kg. per hectare, as mentioned above), but also upon the application method which is to be used. Wettable powders and liquid concentrates are usually applied as aqueous sprays and applied at application rates varying from about 10 to 1500 liters per hectare. With ground equipment, the application rate will generally be in the range of 100 to 500 liters per hectare, whereas aerial spray equipment will generally apply 15 to 80 liters per hectare.

It is preferred to utilize the hydroxyalkanoate compounds in the present invention in an emulsifiable concentrate (EC) to allow for maximum flexibility for both pre-emergence and post-emergence applications. Emulsifiable concentrate formulations are easy for growers to use and provide maximum activity for the compounds in pre-emergence and post-emergence applications because of the uniformity of ground and foliar coverage. This type of formulation provides a thin, monomolecular barrier of chemical causing a maximum surface area for chemical and weed contact. On the soil surface the chemical is available to control weeds germinating from various depths in the soil. The same principle occurs in post-emergence applications as the emulsifiable concentrate form enables excellent foliar coverage of hard to control weeds like cocklebur which have smooth, waxy leaves.

The compounds of the present invention can be applied by pre-emergence/preplant incorporation technique or by post-emergence application. The compounds can be applied as an early post-emergence application, e.g., when the crop/weed growth begins to emerge with one true leaf or first trifoliate leaf above the ground, to growth of the plants of about 10 cm. in height. By such application the residual pre-emergence effects are most advantageously utilized. The compounds have also

exhibited superior activity when applied by late post-emergence application when weeds are mature and most difficult to control. Such later post-emergence application occurs when the plants reach a height greater than about 10 cm.

One of the compounds of and for the present invention, ethoxycarbonylmethyl 5-[2-chloro-4-(trifluoromethyl) phenoxy]-2-nitrobenzoate, hereinafter Compound No. 2, has been evaluated in replicated field test plots of about 3 × 8 m. for pre-emergence, preplant incorporation and post-emergence activity. Also evaluated in these tests were commercially available pre-emergence/preplant incorporation herbicides Sencor, Lorox, Lasso and Modown. In the post-emergence tests, the commercial post-emergence herbicides Basagran and Blazer were evaluated. Compound No. 2 in all of these tests were applied as a 24 Og/litre emulsifiable concentrate formulation.

In the pre-emergence and preplant incorporation trials, the test plots were preseeded with broadleaf weeds (morningglory, velvet-leaf, and wild mustard), with grass weeds (foxtail, millet and green foxtail) and with soybeans. Compound No. 2 was applied in both trials at various rates from 0.28 to 1.12 kg/hA. in 10 litres of water per acre using a calibrated bicycle sprayer with a 150 cm. boom. The pre-emergence treatments were made uniformly to the soil surface on the same day but after the weeds and soybeans were planted. The preplant incorporation treatments were applied to the soil and incorporated uniformly in the top inch of soil using a Lely Roterra before the soybeans and weeds were planted.

Compound No. 2 provided excellent control of the grass weeds at 0.28 kg/hA on pre-emergence trials. Control of most broadleaf and grass weeks was also excellent at rates of 0.56 kg/hA and above with good soybean tolerance. The preplant incorportion application of Compound No. 2 gave very good weed control but somewhat less than that provided by the pre-emergence application. In the pre-emergence and preplant incorporation tests, Compound No. 2 at 0.56 kg/hA performed as well or better than each of the commercial herbicides tested at their respective recommended rates.

Two post-emergence tests were carried out. In both tests, cocklebur, morningglory, velvetleaf and soybeans were seeded. In the first test, the post-emergence applications were made over the top of the soybeans and weeds when the weeds were 5 to 15 cm. tall. In the second test, the post-emergence applications were made when the weeds attained a 15 to 25 cm. height. Weed control ratings were made two and four weeks after each application.

In the first test where Compound No. 2 were applied post-emergence to the weeds two to six inches high, commercial level control was achieved at 0.28 kg/hA and above. In that test, soybean tolerance to Compound No. 2 was good. An unusual observation was made in the plots seven weeks after treatment and three weeks after the last scheduled rating. Plots treated with all rates had very few weeds in them while plots treated with the commercially available post-emergence soybean herbicides showed weed growth and severe soybean competition from weeds that had germinated after treatment and which were not present at the time of application. This shows that Compound No. 2 exhibited excellent early post-emergence weed control with subsequent pre-emergence activity at rates of 0.28 to 0.56 kg/hA. It was concluded that Compound No. 2 controlled late emerging weeds or weed flushes by pre-emergence activity from a post-emergence application used to control the early weed populations.

In the second test where Compound No. 2 was applied by late post-emergence application, Compound No. 2 gave complete control of 15 to 25 cm. weeds treated at 1.12 kg/hA (lower rates were not used because of limited sample). The commercial post-emergence herbicides at the same rate provided only fair control of these older weeds which are generally much tougher to control.

Preferred compounds of the present invention fall within the following formula:

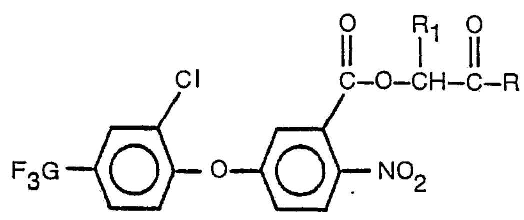

(II)

wherein $R_1$ is selected from the group consisting of hydrogen and an alkyl group of 1—4 carbon atoms; and R is an alkoxy group of 1—4 carbon atoms. Some specific preferred compounds are:

| Compound Number | $R_1$ | R | Name | m.p.°C |
|---|---|---|---|---|
| 1 | H | $CH_3$—O | (Methoxycarbonylmethyl) 5-[2-chloro-4-(trifluoromethyl) phenoxy]-2-nitrobenzoate. | m.p. 102—103°C) |
| 2 | H | $C_2H_5$—O | (Ethoxycarbonylmethyl) 5-[2-chloro-4-(trifluoromethyl) phenoxy]-2-nitrobenzoate. | m.p. 58 |
| 3 | $CH_3$ | $CH_3$—O | (Methoxycarbonyl-1-ethyl) 5-[2-chloro-4-(trifluoromethyl) phenoxy]-2-nitrobenzoate. | oil |
| 4 | $CH_3$ | $C_2H_5$—O | (Ethoxycarbonyl-1-ethyl) 5-[2-chloro-4-(trifluoromethyl) phenoxy]-2-nitrobenzoate. | m.p. 46—49°C |
| 5 | $C_2H_5$ | $C_2H_5$—O | (Ethoxycarbonyl-1-propyl) 5-[2-chloro-4-(trifluoromethyl) phenoxy]-2-nitrobenzoate. | oil |
| 6 | H | n-$C_3H_7$—O | (n-Propyloxycarbonylmethyl) 5-[2-chloro-4-(trifluoromethyl) phenoxy]-2-nitrobenzoate. | m.p. 42—44°C |
| 7 | H | n-$C_4H_9$—O | (n-Butyloxycarbonylmethyl) 5-[2-chloro-4-(trifluoromethyl) phenoxy]-2-nitrobenzoate. | m.p. 42—44°C |
| 8 | H | t-$C_4H_9$—O | (t-Butyloxycarbonylmethyl) 5-[2-chloro-4-(trifluoromethyl) phenoxy]-2-nitrobenzoate. | oil |
| 9 | $CH_3$ (L isomer) | $C_2H_5$—O | (ethoxycarbonyl-1(L)-ethyl) 5-[2-chloro-4-(trifluoromethyl) phenoxy]-2-nitrobenzoate. | oil |

Preparation of ethoxycarbonyl methyl 5-[2-chloro-4-(trifluoromethyl) phenoxy]-2-nitrobenzoate:

To a stirred solution of ethyl glycolate (83.7 g, 0.8 mole) and 5-[2-chloro-4-(trifluoromethyl) phenoxy]-2-nitrobenzoyl chloride (306 g, 0.8 mole) in toluene (800 ml) cooled in an ice bath was added triethylamine (80.8 g, 0.8 mole) dropwise. The exotherm was controlled below 35°C during addition. The reaction temperature was raised to 62° and held for five hours. After cooling the precipitate was filtered. The toluene solution was then washed with 5% sodium hydroxide solution followed by saturated sodium chloride solution. The dried toluene solution was stripped on a rotary evaporator to give 225 g of a brown oil. Vapor phase chromatographic-mass spectral analysis showed that in addition to the desired product there was about 1% of ethyl 5-[2-chloro-4-(trifluoromethyl) phenoxy]-2-nitrobenzoate and about 9% of ethoxycarbonylmethoxycarbonylmethyl 5-[2-chloro-4-(trifluoromethyl) phenoxy]-2-nitrobenzoate. The product can be further purified by crystallization from hexane to give an off-white solid.

m.p. 58—59°C
I.R. (Neat): C, O, 1745 and 1760 $cm^{-1}$
NMR ($CDCl_2$): triplet 1.30 ppm (3H, J = 3.6 Hz);

quartet 4.33 ppm (2H, J = 3.6 Hz);
singlet 4.92 (2H), complex
multiplet 7.1—8.1 ppm (5H) and
doublet 8.21 ppm (1H, J = 4.5 Hz).

A number of compounds of this invention may alternatively be prepared by displacement of an active halogen (e.g. Haloalkyl X) with the salt of an acid

(e.g. 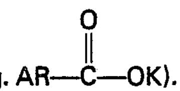).

The following is an example of such a procedure.

To a stirred solution of 85% potassium hydroxide (0.66 g, 0.01 mole) in ethanol (25 ml) was added 5-[2-chloro-4-(trifluoromethyl) phenoxy]2-nitrobenzoic acid. To this mixture was then added ethyl bromoacetate (1.67 g, 0.01 mole) at 30°C. The temperature was raised to 70°C and maintained for 18 hours. The cooled solution was poured into water and the resulting oil solidified to an off-white solid which was filtered and dried to give 3.85 g (86% yield), m.p. 55—6°C.

A still further alternative synthesis is shown by the following reaction scheme:

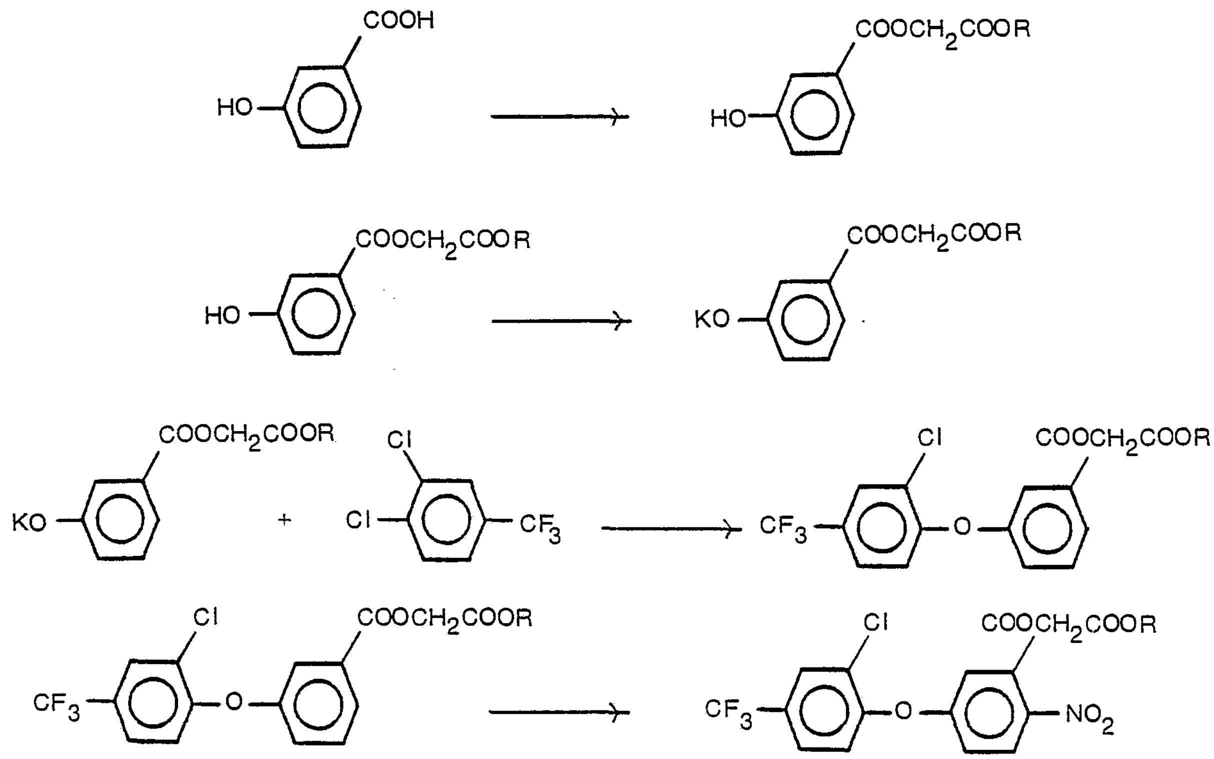

## Claims

1. A herbicidal compound for providing season-long selective weed control by means of post-emergence and pre-emergence herbicidal effects through a single-season treatment of the compound to the cultivated area to be controlled, the compound having the formula:

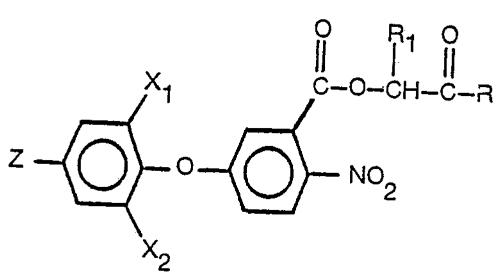

wherein

$X_1$ is a halogen;

$X_2$ is selected from the group consisting of halogen and hydrogen;

Z is a polyhalo$_{1-9}$ alkyl$_{1-4}$ group;

$R_1$ is selected from the group consisting of hydrogen and an alkyl group of 1—4 carbon atoms; and

R is alkoxy of 1—4 carbon atoms,

the following racemic compounds being excluded:

first: Z = $CF_3$; $X_1$ = Cl; $X_2$ = H; $R_1$ = H; R = tertiobutyl oxy
second: Z = $CF_3$; $X_1$ = Cl; $X_2$ = H; $R_1$ = $CH_3$; R = methoxy
third: Z = $CF_3$; $X_1$ = Cl; $X_2$ = H; $R_1$ = $CH_3$; R = ethoxy
fourth: Z = $CF_3$; $X_1$ = Cl; $X_2$ = H; $R_1$ = H; R = ethoxy
fifth: Z = $CF_3$; $X_1$ = Cl; $X_2$ = H; $R_1$ = H; R = methoxy
sixth: Z = $CF_3$; $X_1$ = Br; $X_2$ = Cl; $R_1$ = H; R = ethoxy
seventh: Z = $CCl_3$; $X_1$ = I; $X_2$ = H; $R_1$ = H; R = methoxy
eighth: Z = $CF_3$; $X_1$ = Cl; $X_2$ = Br; $R_1$ = H; R = ethoxy

2. A compound according to claim 1 and of the formula:

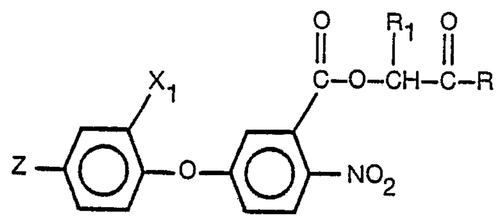

wherein

$X_1$ is chloro;

Z is $CF_3$;

$R_1$ is selected from the group consisting of hydrogen and an alkyl group of 1—4 carbon atoms; and

R is an alkoxy of 1—4 carbon atoms.

3. A compound according to claim 2 where $R_1$ is a hydrogen or a methyl or ethyl groups.

4. A compound according to claim 2 where R is an alkoxy of 1—2 carbon atoms and $R_1$ is hydrogen or a methyl and ethyl group.

5. (Methoxycarbonylmethyl) 5-[2-chloro-4-(trifluoromethyl) phenoxy]-2-nitrobenzoate.

6. (Ethoxycarbonyl-1-propyl) 5-[2-chloro-4-(trifluoromethyl) phenoxy]-2-nitrobenzoate

7. (n-propyloxycarbonylmethyl) 5-[2-chloro-4-(trifluoromethyl) phenoxy]-2-nitrobenzoate.

8. (n-butyloxycarbonylmethyl) 5-[2-chloro-4-(trifluoromethyl) phenoxy]-2-nitrobenzoate.

9. (Ethoxycarbonyl-1(L)-ethyl) 5-[2-chloro-4-(trifluoromethyl) phenoxy]-2-nitrobenzoate.

10. A herbicidal composition for providing season-long selective weed control by means of post-emergence and pre-emergence herbicidal effects through a single season treatment to the cultivated area to be controlled by applying the composition to the area, the composition containing a herbicidally effective amount of a compound as defined in any of claims 1—9.

11. A method for providing season-long selective weed control by means of post-emergence and pre-emergence herbicidal effects through a single-season treatment to the cultivated area to be controlled by applying to the area a composition containing a herbicidally effective amount of a compound having the formula:

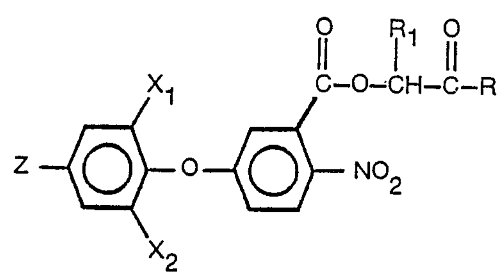

wherein:

$X_1$ is a halogen;

$X_2$ is selected from the group consisting of halogen and hydrogen;
Z is a polyhalo$_{1-9}$ alkyl$_{1-4}$ group;
$R_1$ is selected from the group consisting of hydrogen and an alkyl group of 1—4 carbon atoms; and
R is alkoxy of 1—4 carbon atoms,
the application of this effective amount being made by means of a single treatment, at rates of 0.14 to 0.84 kg/hectare, which is postemergence treatment for the crop and some weeds and which is a preemergence treatment for other late germinating weeds.

12. A method according to claim 11 applying a compound of the formula:

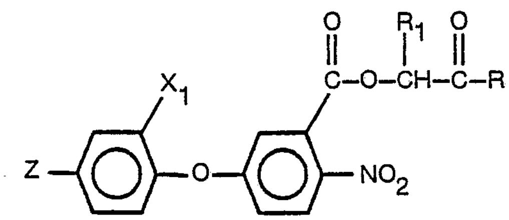

wherein
$X_1$ is chloro;
Z is $CF_3$;
$R_1$ is selected from the group consisting of hydrogen and an alkyl group of 1—4 carbon atoms; and
R is alkoxy of 1—4 carbon atoms.

13. A method according to claim 12 where $R_1$ is a hydrogen or a methyl or ethyl groups.

14. A method according to any of claims 11 to 13, in which the composition is in an emulsifiable concentrate form and composition is applied to the cultivated area by spraying.

15. A method for preparing a compound as defined in claim 1, which comprises reacting a compound of the formula:

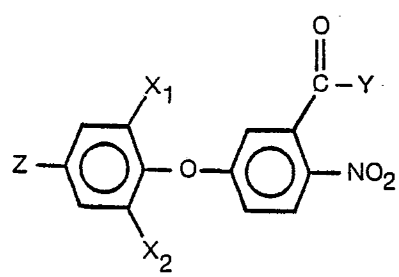

where $X_1$, $X_2$ and Z are as defined in claim 1 and Y is halogen, with a compound of the formula:

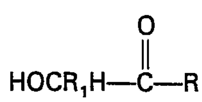

where R and $R_1$ are as defined in claim 1.

16. A method for preparing a compound as defined in claim 1, which comprises reacting a compound of the formula:

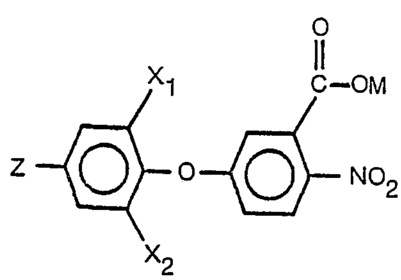

where $X_1$, $X_2$ and Z are as defined in claim 1 and M is a cation, with a compound of the formula:

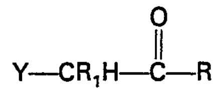

where R and $R_1$ are as defined in claim 1, and Y is halogen.

**Revendications**

1. Un composé herbicide pour assurer un désherbage durant toute une période de culture au moyen d'effets herbicides de postlevée et de prélevée par un traitement, unique au cours de ladite période, de la zone cultivée devant être désherbée, le dit traitement étant effectué à l'aide d'un composé de formule:

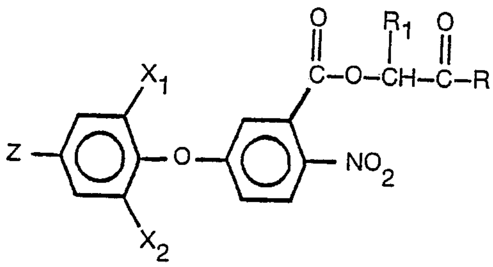

dans laquelle:

$X_1$ est un halogène;

$X_2$ est choisi parmi le groupe se composant d'un halogène et de l'hydrogène;

Z est un groupe polyhalo$_{1-9}$ alkyle$_{1-4}$;

$R_1$ est choisi parmi le groupe se composant de l'hydrogène et d'un groupe alkyle de 1 à 4 atomes de carbone; et

R est alkoxy de 1 à 4 atomes de carbone, les composés racémiques suivants étant exclus:

1*er*: Z = $CF_3$; $X_1$ = Cl; $X_2$ = H; $R_1$ = H; R = tertiobutyloxy.
2*ème*: Z = $CF_3$; $X_1$ = Cl; $X_2$ = H; $R_1$ = $CH_3$; R = méthoxy.
3*ème*: Z = $CF_3$; $X_1$ = Cl; $X_2$ = H; $R_1$ = $CH_3$; R = éthoxy.
4*ème*: Z = $CF_3$; $X_1$ = Cl; $X_2$ = H; $R_1$ = H; R = éthoxy.
5*ème*: Z = $CF_3$; $X_1$ = Cl; $X_2$ = H; $R_1$ = H; R = méthoxy.
6*ème*: Z = $CF_3$; $X_1$ = Br; $X_2$ = Cl; $R_1$ = H; R = éthoxy.
7*ème*: Z = $CCl_3$; $X_1$ = I; $X_2$ = H; $R_1$ = H; R = méthoxy.
8*ème*: Z = $CF_3$; $X_1$ = Cl; $X_2$ = Br; $R_1$ = H; R = éthoxy.

2. Un composé selon la revendication 1 et de formule.

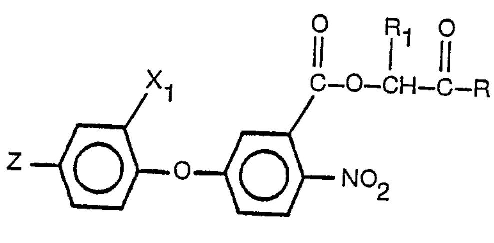

dans laquelle:

$X_1$ est chloro,

Z est $CF_3$,

$R_1$ est choisi parmi le groupe se composant de l'hydrogène et d'un groupe alkyle de 1 à 4 atomes de carbone; et

R est un alkoxy de 1 à 4 atomes de carbone.

3. Un composé selon la revendication 2 où $R_1$ est l'hydrogène ou un groupe méthyle ou éthyle.

4. Un composé selon la revendication 2 où R est un groupe alkoxy de 1 à 2 atomes de carbone et $R_1$ est l'hydrogène ou un groupe méthyle ou éthyle.

5. 5-[2-chloro-4-(trifluorométhyl) phénoxy]-2-nitrobenzoate de méthoxycarbonylméthyle.

6. 5-[2-chloro-4-(trifluorométhyl) phénoxy]-2-nitrobenzoate de éthoxycarbonyl-1-propyle.

7. 5-[2-chloro-4-(trifluorométhyl) phénoxy]-2-nitrobenzoate de n-propyloxycarbonylméthyle.

8. 5-[2-chloro-4-(trifluorométhyl) phénoxy]-2-nitrobenzoate de n-butyloxycarbonylméthyle.

9. 5-[2-chloro-4-(trifluorométhyl) phénoxy]-2-nitrobenzoate de éthoxycarbonyl-1(L)éthyle.

10. Une composition herbicide pour fournir un désherbage durant toute une période au moyen d'effets herbicides de post et prélevée par un traitement unique sur la zone cultivée devant être désherbée en appliquant la composition à la zone, la composition contenant une quantité herbicide efficace d'un composé tel que défini dans l'une des revendications de 1 à 9.

11. Un procédé pour assurer un désherbage durant toute une période au moyen d'effets herbicides de post et prélevée par un traitement unique sur la zone cultivée devant être désherbée en appliquant à la dite zone une composition contenant une quantité herbicide efficace d'une composé de formule:

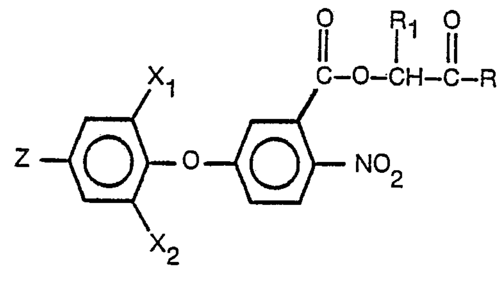

dans laquelle:

$X_1$ est un halogène;

$X_2$ est choisi parmi le groupe se composant de l'hydrogène et des halogènes;

Z est un groupe polyhalo$_{1-9}$ alkyle$_{1-4}$;

$R_1$ est choisi parmi le groupe se composant de l'hydrogène et d'un groupe alkyle de 1 à 4 atomes de carbone; et

R est alkoxy de 1 à 4 atomes de carbone;

l'application de cette quantité efficace étant faite au moyen d'un traitement unique, à des doses de 0,14 à 0,84 kg/ha, le dit traitement étant de postlevée pour la culture et quelques mauvaises herbes et de prélevée pour d'autres mauvaises herbes qui germent tard.

12. Un procédé selon la revendication 11 appliquant un composé de formule:

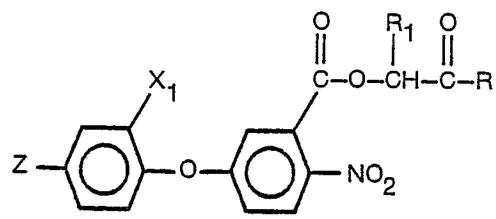

dans laquelle:

$X_1$ est chloro;

Z est $CF_3$;

$R_1$ est choisi parmi le groupe se composant de l'hydrogène et de groupes alkyle de 1 à 4 atomes de carbone; et

R est alkoxy de 1 à 4 atomes de carbone.

13. Un procédé selon la revendication 12 où $R_1$ est l'hydrogène ou un groupe méthyle ou éthyle.

14. Un procédé selon l'une des revendications 11 à 13, dans lequel la composition est sous forme de concentrat émulsionnable et la composition est appliquée à la zone cultivée par pulvérisation.

15. Un procédé pour préparer un composé tel que défini dans la revendication 1, qui comprend la réaction d'un composé de formule:

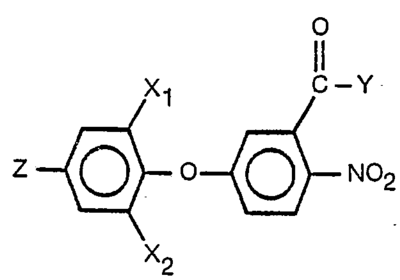

où $X_1$, $X_2$ et Z sont tels que définis dans la revendication 1 et Y est halogène, avec un composé de formule:

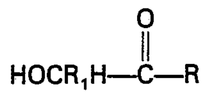

où R et $R_1$ sont tels que définis dans la revendication 1.

16. Un procédé pour préparer un composé tel que défini dans la revendication 1, qui comprend la réaction d'un composé de formule:

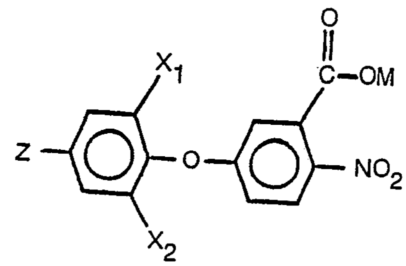

où $X_1$, $X_2$ et Z sont tels que définis dans la revendication 1 et M est un cation, avec un composé de formule:

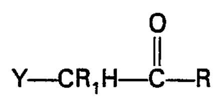

où R et $R_1$ sont tels que définis dans la revendication 1, et Y est halogène.

**Patentansprüche**

1. Herbizide Verbindung für eine selektive Unkrautbekämpfung während der gesamten Wachstumsperiode mit Hilfe herbizider Wirkungen nach dem Auflaufen und vor dem Auflaufen bei einmaliger Behandlung während der Wachstumsperiode der Anbaufläche, die von Unkraut befreit werden soll, wobei die Verbindung der Formel

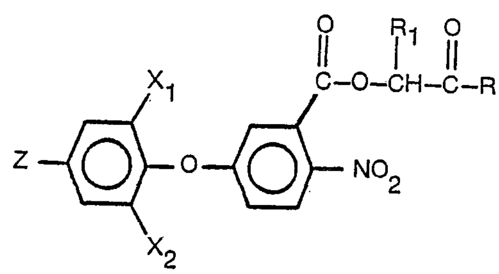

entspricht, in der

$X_1$ ein Halogen ist;

$X_2$ aus der Gruppe bestehend aus Halogen und Wasserstoff ausgewählt ist;

Z eine Polyhalogen$_{1-9}$alkyl$_{1-4}$gruppe ist;

$R_1$ aus der Gruppe bestehend aus Wasserstoff und Alkylgruppen mit 1 bis 4 Kohlenstoffatomen ausgewählt ist; und

R (eine) Alkoxy(gruppe) mit 1 bis 4 Kohlenstoffatomen ist,

wobei folgende racemische Verbindungen ausgenommen sind:
erstens: $Z = CF_3$; $X_1 = Cl$; $X_2 = H$; $R_1 = H$; R = tert.-butyloxy
zweitens: $Z = CF_3$; $X_1 = Cl$; $X_2 = H$; $R_1 = CH_3$; R = Methoxy
drittens: $Z = CF_3$; $X_1 = Cl$; $X_2 = H$; $R_1 = CH_3$; R = Ethoxy
viertens: $Z = CF_3$; $X_1 = Cl$; $X_2 = H$; $R_1 = H$; R = Ethoxy
fünftens: $Z = CF_3$; $X_1 = Cl$; $X_2 = H$; $R_1 = H$; R = Methoxy
sechstens: $Z = CF_3$; $X_1 = Br$; $X_2 = Cl$; $R_1 = H$; R = Ethoxy
siebentens: $Z = CCl_3$; $X_1 = I$; $X_2 = H$; $R_1 = H$; R = Methoxy
achtens: $Z = CF_3$; $X_1 = Cl$; $X_2 = Br$; $R_1 = H$; R = Ethoxy

2. Verbindung nach Anspruch 1 der Formel

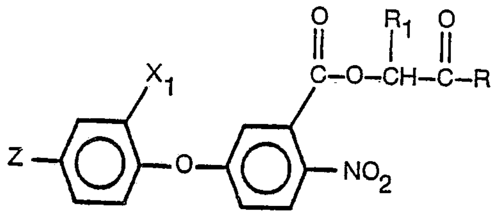

in der

$X_1$ Chlor ist;

Z $CF_3$ ist;

$R_1$ aus der Gruppe bestehend aus Wasserstoff und einer Alkylgruppe mit 1 bis 4 Kohlenstoffatomen ausgewählt ist; und

R (eine) Alkoxy(gruppe) mit 1 bis 4 Kohlenstoffatomen ist.

3. Verbindung nach Anspruch 2, bei der $R_1$ ein Wasserstoff (atom) oder eine Methyl- oder Ethylgruppe ist.

4. Verbindung nach Anspruch 2, bei der R eine Alkoxy (gruppe) mit 1 oder 2 Kohlenstoffatomen und $R_1$ Wasserstoff oder eine Methyl- oder Ethylgruppe ist.

5. (Methoxycarbonylmethyl)-5-[2-chlor-4-(trifluormethyl)-phenoxy]-2-nitrobenzoat.

6. (Ethoxycarbonyl-1-propyl)-5-[2-chlor-4-(trifluormethyl)-phenoxy]-2-nitrobenzoat.

7. (n-Propyloxycarbonylmethyl)-5-[2-chlor-4-(trifluormethyl)-phenoxy]-2-nitrobenzoat.

8. (n-Butyloxycarbonylmethyl)-5-[2-chlor-4-(trifluormethyl)-phenoxy]-2-nitrobenzoat.

9. (Ethoxycarbonyl-1-(L)-ethyl)-5-[2-chlor-4-(trifluormethyl)-phenoxy]-2-nitrobenzoat.

10. Herbizides Mittel für selektive Unkrautbekämpfung während einer gesamten Wachstumsperiode mit Hilfe von herbiziden Wirkungen nach dem Auflaufen und vor dem Auflaufen bei einmaliger Behandlung der Anbaufläche, die von Unkraut befreit werden soll, während der Wachstumsperiode durch Aufbringen des Mittels auf die Fläche, wobei das Mittel eine herbizid wirksame Menge einer Verbindung nach einem der Ansprüche 1 bis 9 enthält.

11. Verfahren für (eine) selektive Unkrautbekämpfung während der gesamten Wachstumsperiode mit Hilfe von herbiziden Wirkungen nach dem Auflaufen und vor dem Auflaufen bei einmaliger Behandlung der Anbaufläche, die von Unkraut befreit werden soll, während der Wachstumsperiode durch Aufbringen auf die Fläche eines Mittels, das eine herbizid wirksame Menge einer Verbindung der Formel

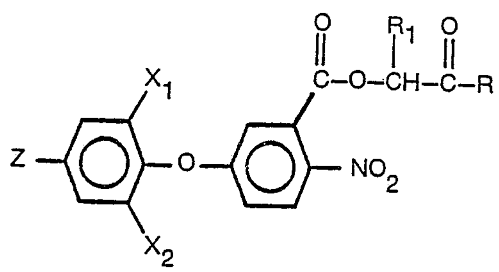

enthält, in der

$X_1$ ein Halogen ist;

$X_2$ aus der Gruppe bestehend aus Halogen und Wasserstoff ausgewählt ist;

Z eine Polyhalogen$_{1-9}$alkyl$_{1-4}$gruppe ist;

$R_1$ aus der Gruppe bestehend aus Wasserstoff und Alkylgruppen mit 1 bis 4 Kohlenstoffatomen ausgewählt ist; und

R (eine) Alkoxy(gruppe) mit 1 bis 4 Kohlenstoffatomen ist,

und daß das Aufbringen dieser wirksamen Menge in Aufwandsmengen von 0,14 bis 0,84 kg je Hektar durch eine einmalige Behandlung erfolgt, die eine Nachauflaufbehandlung für die Nutzpflanzen und einige Unkräuter und eine Vorauflaufbehandlung für andere, spät auskeimende Unkräuter ist.

12. Verfahren nach Anspruch 11, bei dem eine Verbindung der Formel

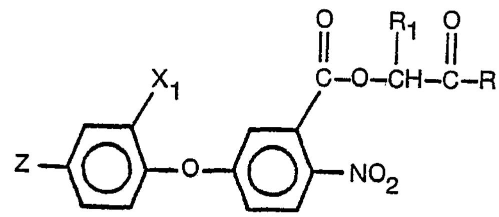

aufgebracht wird, in der

$X_1$ Chlor ist;

Z $CF_3$ ist;

$R_1$ aus der Gruppe bestehend aus Wasserstoff und einer Alkylgruppe mit 1 bis 4 Kohlenstoffatomen ausgewählt ist; und

R eine Alkoxy(gruppe) mit 1 bis 4 Kohlenstoffatomen ist.

13. Verfahren nach Anspruch 12, wobei $R_1$ ein Wasserstoff (atom) oder eine Methyl- oder Ethylgruppe ist.

14. Verfahren nach einem der Ansprüche 11 bis 13, bei dem das Mittel ein emulgierbares Konzentrat ist und durch Spritzen auf die Anbaufläche aufgebracht wird.

15. Verfahren zur Herstellung einer Verbindung nach Anspruch 1, dadurch gekennzeichnet, daß man eine Verbindung der Formel

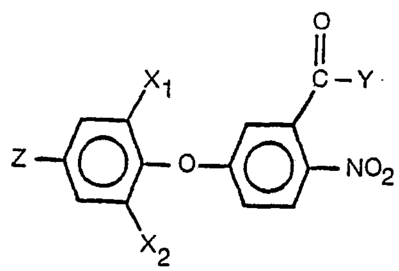

in der $X_1$, $X_2$ und Z wie im Anspruch 1 definiert sind und Y Halogen ist, mit einer Verbindung der Formel

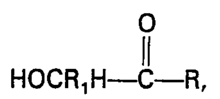

umsetzt, in der R und $R_1$ wie im Anspruch 1 definiert sind.

16. Verfahren zur Herstellung einer Verbindung nach Anspruch 1, dadurch gekennzeichnet, daß man eine Verbindung der Formel

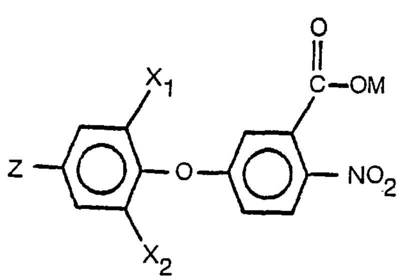

in der $X_1$, $X_2$ und Z wie im Anspruch 1 definiert sind und M ein Kation ist, mit einer Verbindung der Formel

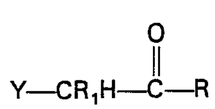

umsetzt, in der R und $R_1$ wie im Anspruch 1 definiert sind und Y Halogen ist.